# EUROPEAN PATENT SPECIFICATION

(11) **EP 0 583 310 B1**
(45) Date of publication and mention of the grant of the patent: **02.10.1996**
(21) Application number: 92909639.4
(22) Date of filing: 10.04.1992
(51) Int. Cl.: D21C 9/10, D21C 9/00, D21C 5/02

(54) **USE OF CELLULASE FOR PULP TREATMENT**
VERWENDUNG VON CELLULASE BEI DER ZELLSTOFFBEHANDLUNG
UTILISATION DE LA CELLULASE POUR LE TRAITEMENT DE LA PATE A PAPIER

(30) Priority: 22.04.1991 DK 738/91
(43) Date of publication of application: 23.02.1994
(73) Proprietor: NOVO NORDISK A/S, 2880 Bagsvaerd (DK)
(72) Inventor: LECLERC, Marc, F-92260 Fontenay-aux-Roses (FR)
(86) International application number: DK9200114
(87) International publication number: WO9218688

(56) References cited:
- EP-A- 0 262 040
- WO-A-89/09259
- WO-A-91/10732
- WO-A-91/17243

## Description

### FIELD OF THE INVENTION

The present invention relates to the use of a cellulase preparation in the treatment of paper pulp.

### BACKGROUND OF THE INVENTION

In the preparation of pulp for paper making, the drainage properties of the pulp may in some cases be unsatisfactory, whereby the capacity of the paper line may be reduced. The drainage properties of the pulp are commonly determined by the Schopper-Riegler test (a high SR value indicating poor drainage) or by Canadian Standard Freeness (a low CSF value indicating poor drainage). Unsatisfactory drainage may particularly occur in case of repulping material that has already been through a pulping and drying process, e.g. dried virgin pulp, recycled fibre or waste paper.

EP-A-262,040 (Cellulose du Pin) and EP-A-351,655 (Cultor) describe improved drainability by use of cellulase and hemicellulase during pulping. JP-A 59-9299 and JP-A 63-59494 describe the use of cellulase during pulping at a high pH (above 9) to improve ink removal from waste paper.

Kraft pulping, a process widely used in the pulp and paper industry, involves alkaline sulfate cooking of the pulp to remove 95% of the lignin. The remaining 5% of the lignin gives the pulp a dark brown colour which tends to get darker in UV light or by oxidation. In order to obtain a white pulp for high quality paper, the brown colour is removed by a multi-stage bleaching process using chlorine or chlorine dioxide.

To reduce the amount of chlorine used in the bleaching process, it has been suggested to subject the pulp to enzymatic treatment with xylanases (cf. K.E.L. Eriksson, Wood Science and Technology 24, 1990, pp. 79-101; M.G. Paice et al., Biotechnol. and Bioeng. 32, 1988, pp. 235-239; and J.C. Pommier et al., Tappi Journal, 1989, pp. 187-191).

### SUMMARY OF THE INVENTION

It has been found that a cellulase preparation exhibiting a high degree of endoglucanase activity may advantageously be used for the treatment of paper pulp, in particular for use in a bleaching, beating or de-inking process or for improving the drainage properties of the pulp.

Accordingly, the present invention as described in claim 1, relates to the use of a cellulase preparation with a high content of endoglucanase and little or no cellobiohydrolase for the treatment of paper pulp.

In the present context, the term "endoglucanase" is understood to indicate a cellulase which attacks amorphous regions of low crystallinity in cellulose fibres resulting in substantially no loss of crystallinity.

### DETAILED DESCRIPTION OF THE INVENTION

According to the invention as described in claim 1, it has been found that when the content of endoglucanase in the cellulase preparation used in the pulping process is high, the damage to cellulose fibres in the pulps is less than when a cellulase preparation is used which has a significant content of cellobiohydrolase. Thus, it is preferred to employ a cellulase preparation which contains at least 50%, in particular at least 90%, (by weight of the total cellulase protein content) of endoglucanase.

Several endoglucanases are known and a selection is used according to the invention. Microbial endoglucanases are preferred for reasons of economy. The endoglucanase should be active and stable at the conditions, especially the pH, of the pulping process. The selected endoglucanases are those derived from Aspergillus (particularly A. niger), Trichoderma (particularly T. viride, T. reesei and T. koningii), Humicola (particularly H. insolens, see US-A-4,435,307), Fusarium, in particular Fusarium oxysporum, Myceliophthora, Phanerochaete, Penicillium, and Geotricum. The endoglucanase included in the cellulase preparation is a monocomponent endoglucanase, and is more preferably one which includes a cellulose-binding domain.

A particularly preferred endoglucanase for use according to the invention is an endoglucanase which is immunoreactive with an antibody raised against a highly purified -43 kD endoglucanase derived from Humicola insolens, DSM 1800. An example of such an endoglucanase is one which has amino acid sequence shown in the appended Sequence Listing ID#1, or a homologue thereof exhibiting endoglucanase activity. In the present context, the term "homologue" is intended to indicate a polypeptide encoded by DNA which hybridizes to the same probe as the DNA coding for the aforesaid endoglucanase under certain specified conditions (such as presoaking in 5xSSC and prehybridizing for 1 h at ∼40°C in a solution of 20% formaminde, 5xDenhardt's solution, 50 mM sodium phosphate, pH 6.8, and 50 µg of denatured sonicated calf thymus DNA, followed by hybridization in the same solution supplemented with 100 µM ATP for 18 h at -40°C). The isolation and preparation of this enzyme is described in the Applicant's European patent EP-B1-0 531 372 granted on 15.02.95 which corresponds to WO-A-91/17243.

The use according to the invention can be applied to any pulp to aid in the bleaching, beating or de-inking thereof, or to improve its drainage properties, provided that this use is not for improving the drainage properties of pulp as a result of treating paper pulp with a cellulase preparation consisting essentially of a homogenous endoglucanase component which is active between pH 6.5 and 9, and which is immunoreactive with an antibody raised against a highly purified ∼43 kD endoglucanase derived from *Humicola insolens,* DSM 1800, or with an endoglucanase having the amino acid sequence shown in the appended Sequence Listing ID# 1 or a homologue thereof exhibiting endoglucanase activity. The disclaimed use is patented in the Applicant's European patent EP-B1-0 531 372 granted on 15.02.95.

The use according to the invention is particularly of interest for pulps with a SR value above 25, and particularly for repulping of previously pulped and dried material such as dried virgin pulp, recycled fibres and waste paper. Other types of pulp which may advantageously be treated with the cellulase preparation are kraft pulp, sulphite pulp or thermomechanical pulp and other high yield pulps.

The Schopper-Riegler number (SR) is determined according to ISO standard 5267 (part 1) on a homogenous pulp with a consistency of 2 g/l. A known volume of pulp is allowed to drain through a metal sieve into a funnel. The funnel is provided with an axial hole and a side hole. The volume of filtrate that passes through the side hole is measured in a vessel graduated in Schopper-Riegler units.

Depending e.g. on the use of recycled water, the process may be acidic (e.g. pH 4 - 7) where an Aspergillus, Phanerochaete, Penicillium, Geotricum or Trichoderma endoglucanase is preferred. Or the process pH may be near-neutral (e.g. pH 6.5-9) where a Humicola, Fusarium or Myceliophthora endoglucanase is preferred.

A suitable cellulase dosage will usually correspond to a cellulase activity at pH 6 of 100 - 10,000 EGU (as defined below) per kg of dry pulp. Where the pulping process is at alkaline pH (above 7), the cellulase dosage should correspond to a cellulase activity at pH 9 of 100 - 10,000 CEVU (as defined below) per kg of dry pulp.

Determination of cellulase activitv (EGU): A substrate solution containing 34.0 g/l CMC (Hercules 7 LFD) in 0.1 M phosphate buffer, pH 6.0. The enzyme sample to be analysed is dissolved in the same buffer. 10 ml substrate solution and 0.5 ml enzyme solution are mixed and transferred to a vibration viscosimeter (e.g. MIVI 3000 available from Sofraser, France) thermostated at 40°C. One endoglucanase unit (EGU) is defined as the amount of enzyme that reduces the viscosity to one half under these conditions.

Determination of cellulase activity (CEVU): A substrate solution containing 33.3 g/l CMC (Hercules 7 LFD) in Tris-buffer, pH 9.0, is prepared. The enzyme sample to be determined is dissolved in the same buffer. 10 ml substrate solution and 0.5 ml enzyme solution are mixed and transferred to a viscosimeter (Haake VT 181, NV sensor, 181 rpm) thermostated at 40°C. One Cellulase Viscosity Unit (CEVU) is defined as the amount of enzyme which reduces the viscosity to one half under these conditions.

When the cellulase preparation is to be employed according to the invention for pulping waste paper, de-inking may be achieved by carrying out the pulping at high pH (above 9) in the presence of de-inking chemicals (such as sodium hydroxide, sodium silicate, hydrogen peroxide and surfactant), followed by ink separation (e.g. by flotation and/or rinsing). This embodiment of the invention is particularly advantageous since the cellulase/endoglucanase will also serve to improve the de-inking, concomitantly with improving drainage. At the high pH used for de-inking, it is preferred to use an endoglucanase from Humicola.

The duration of the pulping will generally be 5 - 30 minutes, and this may optionally be followed by maceration (i.e. incubation with or without stirring) to let the enzyme action continue. The temperature throughout this treatment will generally be 15-80°C, typically 30-50°C. The total duration of the cellulase action (i.e. pulping + maceration, if any) will generally be 30-180 minutes.

Pulp prepared according to the invention can be used for conventional paper making.

The invention is further described in the follwing example which is not in any way intended to limit the scope of the invention as claimed.

### EXAMPLE

### Use of ∼43 kD Humicola endoglucanase for the treatment of paper pulp

The ∼43 kD endoglucanase derived from Humicola insolens, DSM 1800, was used for the treatment of several types of paper pulp with a view to investigating the effect of the enzyme on pulp properties.

The used of this particular endoglucanase for improving the drainage properties of pulp is disclaimed.

The experimental conditions were as follows.

### Pulps

1. Waste paper mixture: composed of 33% newsprint, 33% magazines and 33% computer paper. With or without deinking chemicals (WPC or WP, respectively)
2. Recycled cardboard containers (RCC).
3. Bleached kraft: made from pine (BK).
4. Unbleached thermomechanical: made from fir (TMP).

### Enzymatic treatment

A preparation of the -43 kD endoglucanase was diluted to 7 CEVU/ml and added to each of the pulps indicated above (50 g DS, consistency 3%). The enzyme dose was 2400 CEVU/kg dry pulp. The enzymatic treatment was conducted at a pH of 7.5 and at 40°C with gentle stirring for 60 minutes. A sample was taken after 30 minutes to monitor the progression of the reaction.

After 60 minutes, the pulp was diluted to a consistency of 0.5% with cold water (+4°C) in order to stop the reaction.

Drainage of the wet pulp was determined as described above and given Schopper-Riegler (SR) values. The drainage time (DT) under vacuum was also determined.

The results are summarized in the following tables

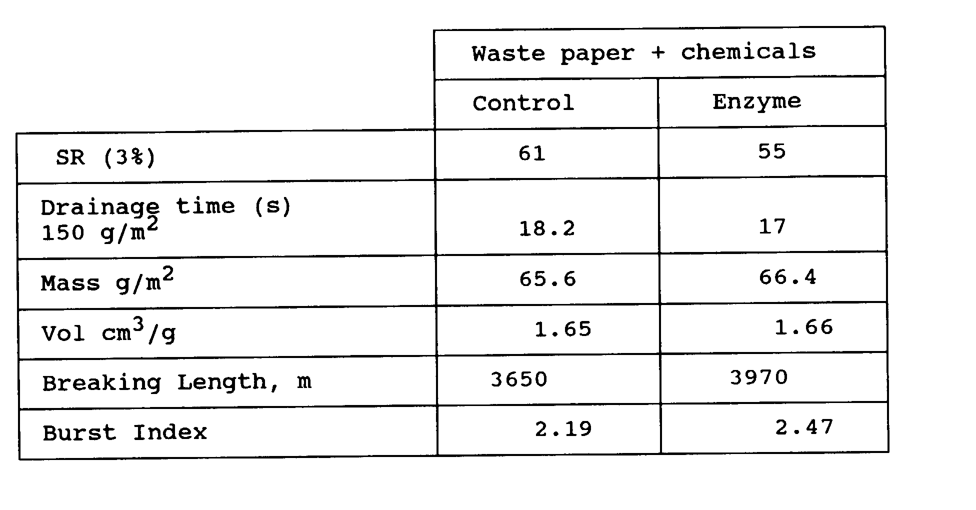

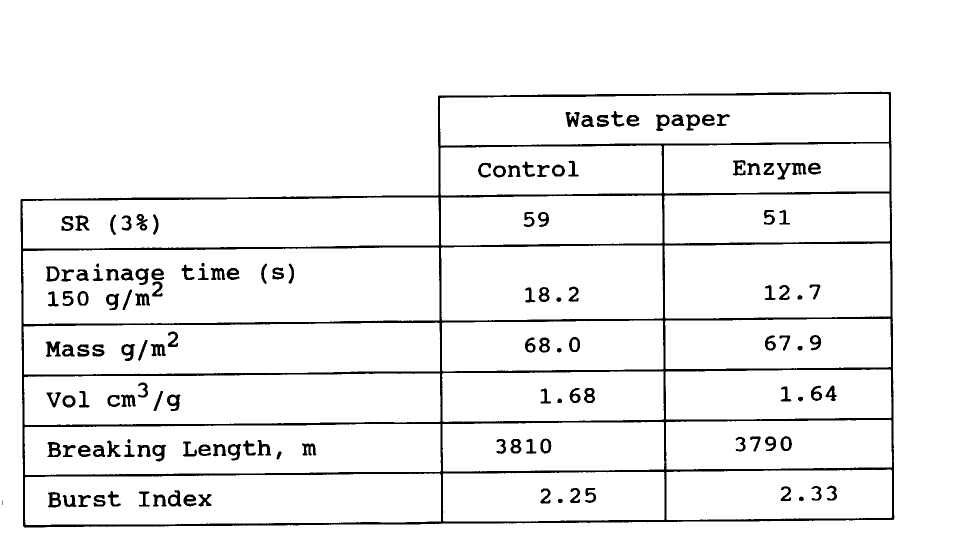

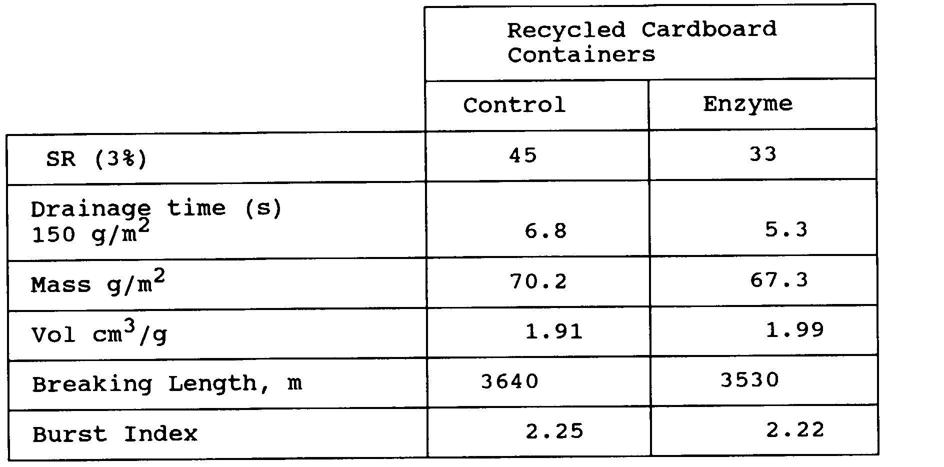

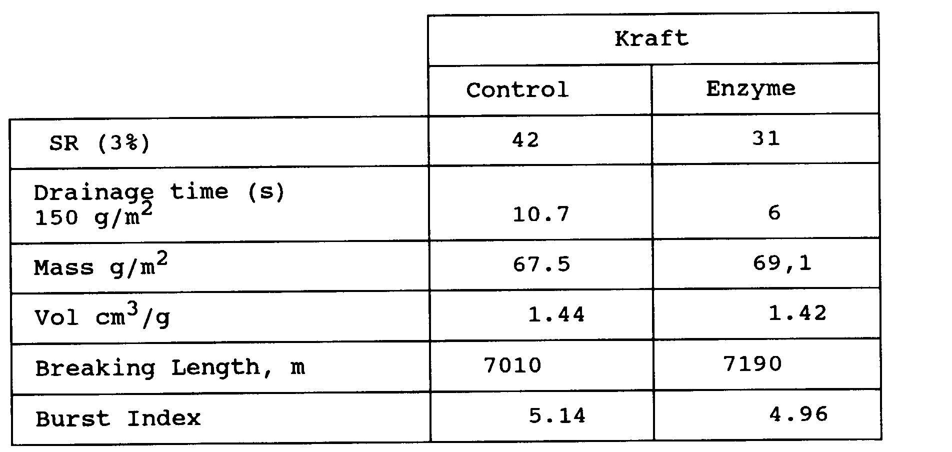

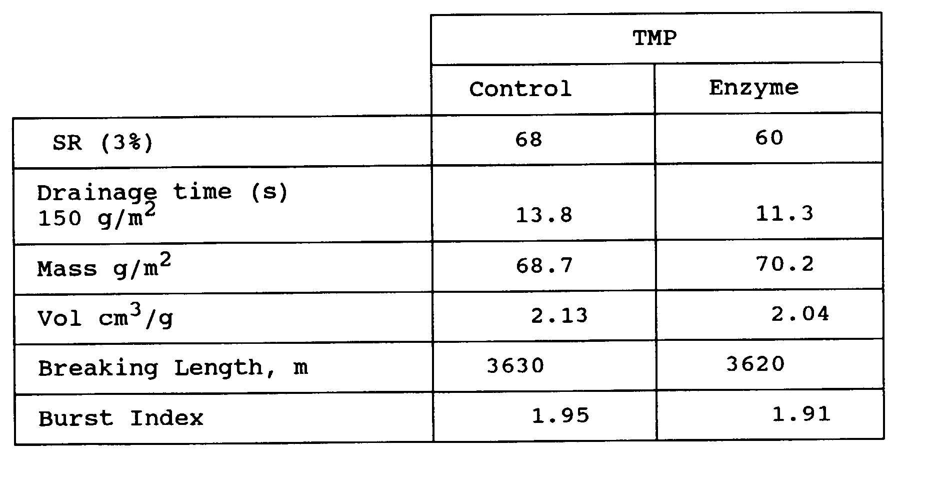

Control experiments. Same conditions as the enzyme treatment, except no enzyme addition.

It appears from the table that the -43 kD endoglucanase treatment causes a significant decrease in SR values and significantly improves drainage of pulps used in papermaking.

Paper sheets were made from the various pulps on a Rapid Köthen device and measured for strength according to different parameters (including breaking length). No decrease in strength properties due to enzyme action was observed, as shown in the table.

### SEQUENCE LISTING

(I) GENERAL INFORMATION:
   (i) APPLICANT: NOVO NORDISK A/S,
   (ii) TITLE OF INVENTION:
   (iii) NUMBER OF SEQUENCES: 1
   (iv) CORRESPONDENCE ADDRESS:
      (A) ADDRESSEE: NOVO NORDISK A/S, Patent Department
      (B) STREET: Novo Alle
      (C) CITY: Bagsvaerd
      (E) COUNTRY: DENMARK
      (F) ZIP: DK-2880
   (v) COMPUTER READABLE FORM:
      (A) MEDIUM TYPE: Floppy disk
      (B) COMPUTER: IBM PC compatible
      (C) OPERATING SYSTEM: PC-DOS/MS-DOS
      (D) SOFTWARE: PatentIn Release #1.0, Version #1.25
   (vi) CURRENT APPLICATION DATA:
      (A) APPLICATION NUMBER:
      (B) FILING DATE:
      (C) CLASSIFICATION:
   (viii) ATTORNEY/AGENT INFORMATION:
      (A) NAME: Thalsoe-Madsen, Birgit
   (ix) TELECOMMUNICATION INFORMATION:
      (A) TELEPHONE: +45 4444 8888
      (B) TELEFAX: +45 4449 3256
      (C) TELEX: 37304
(2) INFORMATION FOR SEQ ID NO:1:
   (i) SEQUENCE CHARACTERISTICS:
      (A) LENGTH: 1060 base pairs
      (B) TYPE: nucleic acid
      (C) STRANDEDNESS: single
      (D) TOPOLOGY: linear
   (ii) MOLECULE TYPE: cDNA
   (iii) HYPOTHETICAL: NO
   (vi) ORIGINAL SOURCE:
      (A) ORGANISM: Humicola insolens
      (B) STRAIN: DSM 1800
   (ix) FEATURE:
      (A) NAME/KEY: mat_peptide
      (B) LOCATION: 73..927
   (ix) FEATURE:
      (A) NAME/KEY: sig_peptide
      (B) LOCATION: 10..72
   (ix) FEATURE:
      (A) NAME/KEY: CDS
      (B) LOCATION: 10..927
   (xi) SEQUENCE DESCRIPTION: SEQ ID NO:1:

GGATCCAAG ATG CGT TCC TCC CCC CTC CTC CCG TCC GCC GTT GTG GCC 48
Met Arg Ser Ser Pro Leu Leu Pro Ser Ala Val Val Ala
-21 -20 -15 -10

GCC CTG CCG GTG TTG GCC CTT GCC GCT GAT GGC AGG TCC ACC CGC TAC 96
Ala Leu Pro Val Leu Ala Leu Ala Ala Asp Gly Arg Ser Thr Arg Tyr
-5 1 5

TGG GAC TGC TGC AAG CCT TCG TGC GGC TGG GCC AAG AAG GCT CCC GTG 144
Trp Asp Cys Cys Lys Pro Ser Cys Gly Trp Ala Lys Lys Ala Pro Val
10 15 20

AAC CAG CCT GTC TTT TCC TGC AAC GCC AAC TTC CAG CGT ATC ACG GAC 192
Asn Gln Pro Val Phe Ser Cys Asn Ala Asn Phe Gln Arg Ile Thr Asp
25 30 35 40

TTC GAC GCC AAG TCC GGC TGC GAG CCG GGC GGT GTC GCC TAC TCG TGC 240
Phe Asp Ala Lys Ser Gly Cys Glu Pro Gly Gly Val Ala Tyr Ser Cys
45 50 55

GCC GAC CAG ACC CCA TGG GCT GTG AAC GAC GAC TTC GCG CTC GGT TTT 288
Ala Asp Gln Thr Pro Trp Ala Val Asn Asp Asp Phe Ala Leu Gly Phe
60 65 70

GCT GCC ACC TCT ATT GCC GGC AGC AAT GAG GCG GGC TGG TGC TGC GCC 336
Ala Ala Thr Ser Ile Ala Gly Ser Asn Glu Ala Gly Trp Cys Cys Ala
75 80 85

TGC TAC GAG CTC ACC TTC ACA TCC GGT CCT GTT GCT GGC AAG AAG ATG 384
Cys Tyr Glu Leu Thr Phe Thr Ser Gly Pro Val Ala Gly Lys Lys Met
90 95 100

GTC GTC CAG TCC ACC AGC ACT GGC GGT GAT CTT GGC AGC AAC CAC TTC 432
Val Val Gln Ser Thr Ser Thr Gly Gly Asp Leu Gly Ser Asn His Phe
105 110 115 120

GAT CTC AAC ATC CCC GGC GGC GGC GTC GGC ATC TTC GAC GGA TGC ACT 480
Asp Leu Asn Ile Pro Gly Gly Gly Val Gly Ile Phe Asp Gly Cys Thr
125 130 135

CCC CAG TTC GGC GGT CTG CCC GGC CAG CGC TAC GGC GGC ATC TCG TCC 528
Pro Gln Phe Gly Gly Leu Pro Gly Gln Arg Tyr Gly Gly Ile Ser Ser
140 145 150

CGC AAC GAG TGC GAT CGG TTC CCC GAC GCC CTC AAG CCC GGC TGC TAC 576
[?]rg Asn Glu Cys Asp Arg Phe Pro Asp Ala Leu Lys Pro Gly Cys Tyr
155 160 165

TGG CGC TTC GAC TGG TTC AAG AAC GCC GAC AAT CCG AGC TTC AGC TTC 624
Trp Arg Phe Asp Trp Phe Lys Asn Ala Asp Asn Pro Ser Phe Ser Phe
170 175 180

CGT CAG GTC CAG TGC CCA GCC GAG CTC GTC GCT CGC ACC GGA TGC CGC 672
Arg Gln Val Gln Cys Pro Ala Glu Leu Val Ala Arg Thr Gly Cys Arg
185 190 195 200

CGC AAC GAC GAC GGC AAC TTC CCT GCC GTC CAG ATC CCC TCC AGC AGC 720
Arg Asn Asp Asp Gly Asn Phe Pro Ala Val Gln Ile Pro Ser Ser Ser
205 210 215

ACC AGC TCT CCG GTC AAC CAG CCT ACC AGC ACC AGC ACC ACG TCC ACC 768
Thr Ser Ser Pro Val Asn Gln Pro Thr Ser Thr Ser Thr Thr Ser Thr
220 225 230

TCC ACC ACC TCG AGC CCG CCA GTC CAG CCT ACG ACT CCC AGC GGC TGC 816
Ser Thr Thr Ser Ser Pro Pro Val Gln Pro Thr Thr Pro Ser Gly Cys
23[?] 240 245

ACT GCT GA[?] [?][?][?] TGG GCT CAG TGC GGC GGC AAT GGC TGG AGC GGC TGC 864
Thr Ala Glu Ar[?] Trp Ala Gln Cys Gly Gly Asn Gly Trp Ser Gly Cys
250 255 260

ACC ACC TGC GTC GCT GGC AGC ACT TGC ACG AAG ATT AAT GAC TGG TAC 912
Thr Thr Cys Val Ala Gly Ser Thr Cys Thr Lys Ile Asn Asp Trp Tyr
265 270 275 280

CAT CAG TGC CTG TAGACGCAGG GCAGCTTGAG GGCCTTACTG GTGGCCGCAA 964
His Gln Cys Leu
285

CGAAATGACA CTCCCAATCA CTGTATTAGT TCTTGTACAT AATTTCGTCA TCCCTCCAGG 1024

GATTGTCACA TAAATGCAAT GAGGAACAAT GAGTAC 1060

## Claims

1. Use of a cellulase preparation for the treatment of paper pulp, which preparation comprises a monocomponent endoglucanase derived from an *Aspergillus, Phanerochaete, Penicillium, Geotricum, Trichoderma, Humicola, Fusarium* or *Myceliophthora* strain, and little or no cellobiohydrolase,
provided that the use is not for improving the drainage properties of pulp by treating paper pulp with a cellulase preparation consisting essentially of a homogenous endoglucanase component which is active between pH 6.5 and 9, and which is immunoreactive with an antibody raised against a highly purified -43 kD endoglucanase derived from *Humicola insolens,* DSM 1800, or with an endoglucanase having the amino acid sequence shown in the appended Sequence Listing ID# 1 or a homologue thereof exhibiting endoglucanase activity.

2. The use according to claim 1, wherein the endoglucanase is one which includes a cellulose-binding domain.

3. The use according to claim 1 or 2, wherein the cellulase preparation contains at least 50% (by weight of the total cellulase protein content) of endoglucanase.

4. The use according to claim 3, wherein the cellulase preparation contains at least 90% (by weight of the total cellulase protein content) of endoglucanase.

5. The use according to any of claims 1-4, wherein the endoglucanase has a pH optimum at pH 4 - 7 and is derived from an *Aspergillus, Phanerochaete, Penicillium, Geotricum* or *Trichoderma* strain.

6. The use according to any of claims 1-4, wherein the endoglucanase has a pH optimum at pH 6.5 - 9 and is derived from a *Humicola, Fusarium* or *Myceliophthora* strain.

7. The use according to claim 1, wherein the endoglucanase is one which is immunoreactive with an antibody raised against a highly purified ∼43 kD endoglucanase derived from *Humicola insolens,* DSM 1800.

8. The use according to claim 7, wherein the endoglucanase has the amino acid sequence shown in the appended Sequence Listing ID# 1, or a homologue thereof exhibiting endoglucanase activity.

## Patentansprüche

1. Verwendung eines Cellulase-Präparats zur Behandlung von Papierzellstoff, wobei das Präparat eine Einkomponenten-Endoglucanase enthält, die von einem *Aspergillus-, Phanerochaete-, Penicillium-, Geotricum-, Trichoderma-, Humicola-, Fusarium-* oder *Myceliophthora-Stamm* stammt, und wenig oder keine Cellobiohydrolase,
unter der Voraussetzung, daß die Verwendung nicht zur Verbesserung der Entwässerungseigenschaften von Zellstoff durch Behandlung von Papierzellstoff mit einem Cellulase-Präparat ist, das im wesentlichen aus einer homogenen Endoglucanase-Komponente besteht, die zwischen pH 6,5 und 9 aktiv ist, und die mit einem Antikörper immunreaktiv ist, der gegen eine hochgereinigte ∼43 kD-Endoglucanase erzeugt wurde, die von *Humicola insolens,* DSM 1800, stammt, oder mit einer Endoglucanase mit der Aminosäuresequenz, die im beigefügten Sequenzprotokoll ID# 1 gezeigt ist oder einem Homologen davon, das eine Endoglucanase-Aktivität aufweist.

2. Verwendung nach Anspruch 1, wobei die Endoglucanase eine ist, die eine Cellulose-bindende Domäne einschließt.

3. Verwendung nach Anspruch 1 oder 2, wobei das Cellulase-Präparat mindestens 50% (des Gewichts des gesamten Cellulase-Proteingehalts) an Endoglucanase enthält.

4. Verwendung nach Anspruch 3, wobei das Cellulase-Präparat mindestens 90% (des Gewichts des gesamten Cellulase-Proteingehalts) an Endoglucanase enthält.

5. Verwendung nach einem der Ansprüche 1-4, wobei die Endoglucanase ein pH-Optimum bei pH 4-7 besitzt und von einem *Aspergillus-, Phanerochaete-, Penicillium-, Geotricum-* oder *Trichodenna-Stamm* stammt.

6. Verwendung nach einem der Ansprüche 1-4, wobei die Endoglucanase ein pH-Optimum bei pH 6,5-9 besitzt und von einem *Humicola-, Fusarium-* oder *Myceliophthora-Stamm* stammt.

7. Verwendung nach Anspruch 1, wobei die Endoglucanase eine ist, die mit einem Antikörper immunreaktiv ist, der gegen eine hochgereinigte ∼43 kD-Endoglucanase erzeugt wurde, die von *Humicola insolens,* DSM 1800, stammt.

8. Verwendung nach Anspruch 7, wobei die Endoglucanase die Aminosäuresequenz besitzt, die in dem beigefügten Sequenzprotokoll ID# 1 gezeigt ist, oder ein Homologes davon, das eine Endoglucanase-Aktivität aufweist.

## Revendications

1. Utilisation d'une préparation de cellulase pour le traitement de pâte à papier, préparation qui comprend une endoglucanase monocom-posant dérivée d'une souche *d'Aspergillus,* de *Phanerochaete,* de *Penicillium*, de *Geotricum*, de *Trichoderma*, *d'Humicola*, de *Fusarium* ou de *Myceliophthora*, et peu ou pas de cellobiohydrolase,
dès lors que l'utilisation n'est pas pour l'amélioration de la capacité d'égouttage de la pâte par traitement de la pâte à papier par une préparation de cellulase consistant essentiellement en un composant homogène d'endoglucanase qui est actif entre pH 6,5 et 9, et qui est immuno-réactif avec un anticorps cultivé contre une endoglucanase ∼43 kl) hautement purifiée dérivée *de Humicola insolens,* DSM 1800, ou avec une endoglucanase ayant la séquence d'acides aminés illustrée dans le Listage de Séquence ID#1 annexé ou un homologue de celle-ci présentant une activité d'endoglucanase.

2. L'utilisation selon la revendication 1, dans laquelle l'endoglucanase est une endoglucanase qui comprend un domaine de liaison à la cellulose.

3. L'utilisation selon la revendication 1 ou 2, dans laquelle la préparation de cellulase contient au moins 50 % (en poids du contenu total de protéine cellulase) d'endoglucanase.

4. L'ulilisation selon la revendication 3, dans laquelle la préparation de cellulase contient au moins 90 % (en poids du contenu total de protéine cellulase) d'endoglucanase.

5. L'utilisation selon l'une des revendications 1 à 4, dans laquelle l'Endoglucanase présente un pH optimal à pH 4 à 7 et est dérivée d'une souche d'*Aspergillus*, de *Phanerochaete*, de *Penicillium*, de *Geotricum* ou de *Trichoderma*.

6. L'utilisation selon l'une des revendications 1 à 4, dans laquelle l'endoglucanase présente un pH optimal à pH 6,5 à 9 et est dérivée d'une souche *d'Humicola, de Fusarium* ou de *Myceliophthora.*

7. L'utilisation selon la revendication 1, dans laquelle l'endoglucanase est une endoglucanase qui est immuno-réactive avec un anticorps cultivé contre une endoglucanase ∼43 kD hautement purifiée dérivée de *Humicola insolens*, DMS 1.800.

8. L'utilisation selon la revendication 7, dans laquelle l'endoglucanase présente la séquence d'acides aminés illustrée dans le Listage de Séquence ID#1, ou un homologue présentant une activité d'endoglucanase.
